# FASCICULE DE BREVET EUROPEEN

(11) **EP 1 996 106 B1**
(45) Date de publication et mention de la délivrance du brevet: **30.04.2014**
(21) Numéro de dépôt: 06726075.2
(22) Date de dépôt: 13.03.2006
(51) Int. Cl.: A61B 18/24

(54) **INSTRUMENT LASER, APPLICABLE À L'OCCLUSION VASCULAIRE NOTAMMENT POUR UN TRAITEMENT ENDOVEINEUX AINSI QU'À LA PERFORATION OU À LA DÉTERSION TISSULAIRE**
LASERINSTRUMENT FÜR DEN GEFÄSSVERSCHLUSS, INSBESONDERE FÜR INTRAVENÖSE BEHANDLUNG UND ZUR PERFORATION ODER DETERSION VON GEWEBE
LASER INSTRUMENT FOR VASCULAR OCCLUSION, IN PARTICULAR FOR INTRAVENOUS TREATMENT, AND FOR PERFORATION OR DETERSION OF TISSUE

(43) Date de publication de la demande: 03.12.2008
(73) Titulaire: Anastasie, Bruno, 94210 La Varenne-Saint-Hilaire (FR)
(72) Inventeur: Anastasie, Bruno, 94210 La Varenne-Saint-Hilaire (FR)
(74) Mandataire: Debay, Yves
(86) Numéro de dépôt international: PCT/FR2006/000546
(87) Numéro de publication internationale: WO 2007/104836

(56) Documents cités:
- EP-A2- 1 350 481
- WO-A-01/03596
- WO-A-98/24513
- ES-A1- 2 188 398
- US-A1- 2003 199 860
- US-A1- 2005 131 400

## Description

La présente invention se rapporte à un instrument de traitement par la lumière, notamment à un laser endoveineux pouvant être utilisé pour le traitement des varices.

La phlébectomie est une opération chirurgicale lourde et coûteuse. En outre, elle est génératrice de cicatrices.

Des techniques plus récentes pour la sclérose vasculaire, notamment le traitement des varices, par exemple la technique exposée dans le document US 2003/0078569, si elles sont moins lourdes pour le patient, elles nécessites néanmoins une préparation de cathétérisme, un habillage de l'opérateur, des champs stériles, une grande désinfection et des manipulation complexes. Elles utilisent en outre des fibres optiques longues et coûteuses. De plus, cette technique nécessite l'injection d'un produit susceptible de provoquer des allergies. C'est le cas de la sclérose conventionnelle en cabinet qui utilise des sclérosants chimiques.

Le document US 2005/0131400 détaille un dispositif de conduction d'un laser dans une fibre optique, l'extrémité de la fibre optique traversant un cathéter pour être introduite dans un vaisseau sanguin. Le faisceau laser diffusé à l'extrémité de la fibre optique à l'intérieur d'une veine participe au traitement de varices en intervenant sur les cellules endothéliales de la paroi de la veine. Toutefois, un tel dispositif ne permet pas d'optimiser la diffusion de la lumière lors d'un traitement circonférentiel de la paroi d'un vaisseau.

Le but de l'invention est de proposer une technique plus simple et un matériel, instrument et système, moins coûteux et plus faciles à manipuler.

Ce but est atteint par un instrument selon la revendication 1.

Selon un premier objectif de l'invention, un tel instrument comprend un guide de lumière, par exemple une fibre optique, et un support rigide pour le guide, par exemple une aiguille formant gaine autour du guide de lumière.

Une telle aiguille optique serait reliée à une source laser de longueur d'onde variable par un vecteur de lumière connecté de part et d'autre au laser et à l'aiguille par une prise standard (SMA ou autre), restérilisable chimiquement pour l'utilisation en milieu chirurgical stérile. Ainsi, contrairement aux utilisations précédentes où la souplesse d'une fibre optique est privilégiée, afin par exemple de pouvoir parcourir une veine voire une artère sur une grande distance depuis une entrée de la fibre jusqu'à une zone à traiter, on privilégie au contraire une certaine rigidité qui est apportée par le support. Bien entendu cette rigidité n'est pas absolue, mais plutôt à comparer à la souplesse d'une fibre optique telle qu'utilisée, notamment en laser endoveineux. Cette rigidité peut être nuancée selon l'utilisation. Par exemple dans le cas d'une aiguille biseautée, celle-ci peut être suffisamment rigide pour permettre de piquer au travers de la peau d'un patient.

De préférence, pour le traitement des varices, l'aiguille peut avoir un diamètre extérieur compris dans un intervalle de 0,45 mm à 1 mm. Le guide de lumière peut avoir un diamètre extérieur de 200 à 1000 microns (1 mm).

Avantageusement, l'instrument peut comprendre des moyens de branchement à une source de lumière, notamment à une source de lumière laser. La source de lumière laser peut avoir une longueur d'onde comprise entre 800 et 980nm, qui est adaptée à l'utilisation endovasculaire. D'autres longueurs d'onde sont aussi possibles selon les applications.

L'aiguille peut être droite ou courbe. La longueur de l'aiguille et du guide de lumière peut varier en fonction des applications. Le guide de lumière peut comprendre, par exemple, un habillage intérieur d'une aiguille tubulaire. Cet habillage sera avantageusement réalisé en silice.

En tant que variante, l'instrument peut comprendre une fibre optique en tant que guide de lumière, par exemple une fibre d'un type couramment utilisé pour des applications médicales. Cette fibre peut être montée dans une aiguille tubulaire.

L'instrument peut comprendre des moyens pour une émission sensiblement frontale de la lumière etcomprend des moyens pour une émission latérale de la lumière. La diffusion latérale peut se faire au travers d'une ou plusieurs fenêtres latérales. Selon l'utilisation prévue, les fenêtres peuvent être réparties transversalement ou longitudinalement le long du guide de lumière et/ou du support.

L'instrument peut en outre comprendre un chenal, le reflux du sang dans le chenal attestant de la bonne position intra-vasculaire de l'aiguille ; un chenal peut aussi être prévu pour y injecter un anesthésique.

Selon un deuxième objectif de l'invention, un système de traitement peut comprendre une source de lumière laser. Notamment pour le traitement des varices, la longueur d'onde de cette lumière est avantageusement comprise entre 800 et 1000 nm. Encore plus avantageusement, cette longueur d'onde est de 980 nm, du fait de son absorption privilégiée par l'hémoglobine oxygénée et par l'eau.

un procédé de traitement par lumière laser peut avantageusement utiliser un instrument ou un système selon l'invention. Il peut notamment être utilisé pour des applications vasculaires, notamment pour le traitement de varice. Pour leur traitement, le laser peut être utilisé pour la sclérose de varice. Il peut aussi être utilisé pour l'occlusion artérielle remplaçant alors l'embolisation chimique.

Par exemple après une anesthésie locale, on pique avec l'instrument selon l'invention au droit d'une varice à traiter, au travers de la peau, comme pour une ponction. La piqûre peut se faire sous contrôle de la vue ou sous contrôle échographique. L'instrument peut servir lui-même à l'injection d' anesthésique au travers d'un chenal prévu à cet effet, évitant ainsi une piqûre et l'emploi d'une seringue supplémentaires. Un chenal, éventuellement le même, peut aussi être prévu pour vérifier l'afflux de sang. On peut ainsi vérifier le bon positionnement de l'instrument dans le système vasculaire. Une anesthésie par intumescence péri-vasculaire est pratiquée. Le repérage peut se faire sous échographie.

Lorsque l'instrument est en position, on effectue un tir laser. Il est avantageux d'imprimer une rotation à l'extrémité du guide de lumière afin d'assurer une lésion circonférentielle de la paroi de la varice à traiter. On obtient ainsi une sclérose de la varice, qui n'est alors plus irriguée. On observe en laser endoveineux une photocoagulation du sang avec rétraction tissulaire par contraction du collagène en proportion plus importante dans la veine variqueuse.

On retire ensuite l'instrument, qui peut-être décharbonné avec une compresse stérile. L'instrument est alors prêt pour traiter une autre varice. Une ponction directe sans aiguille de guidage est aussi possible.

La fibre optique utilisée peut être une fibre d'un type courant, notamment d'un type utilisé pour les fibroscopies interventionnelles, laser endoveineux, chirurgie ...

Le coût d'un instrument selon l'invention peut être divisé par cinq ou dix relativement à un instrument actuellement utilisé pour le traitement des varices par lumière laser s'il est fabriqué à grande échelle.

D'autres particularités et avantages de l'invention ressortiront encore de la description ci-après, relative à des exemples non limitatifs.

Aux dessins annexés :
- la figure 1 est une vue schématique d'un système selon l'invention, en cours d'utilisation ;
- la figure 2 est une vue en coupe longitudinale d'un premier mode de réalisation pour une aiguille comprenant un guide optique sous la forme d'une fibre optique et dans lequel la diffusion de la lumière se fait à travers une ouverture axiale ;
- la figure 3 est une coupe transversale selon III de l'aiguille de la figure 2;
- la figure 4 est coupe sur une extrémité d'un guide de lumière adapté pour une diffusion latérale de la lumière, d'un deuxième mode de réalisation ;
- la figure 5 est une coupe transversale, similaire à celle de la figure 2, d'un troisième mode de réalisation, dans lequel le guide de lumière est appliqué sur la paroi interne de l'aiguille, et dans lequel la diffusion de la lumière se fait à travers une ouverture axiale ;
- la figure 6 est une coupe transversale, similaire à celles des figures 2 et 5, d'un troisième mode de réalisation, dans lequel le guide de lumière est appliqué sur la paroi interne de l'aiguille, et dans lequel la diffusion de la lumière se fait à travers une ouverture axiale et des ouvertures latérales;
- la figure 7 est une coupe transversale selon VII de l'aiguille de la figure 5; et,
- la figure 8 est une coupe transversale selon VIII de l'aiguille de la figure 6.

Par la suite, on entend par aiguille une aiguille de type tubulaire, suffisamment rigide pour permettre une manipulation précise du guide de lumière. En outre, notamment pour le traitement endoveineux, cette aiguille doit être suffisamment rigide pour percer la peau, telle une aiguille de ponction.

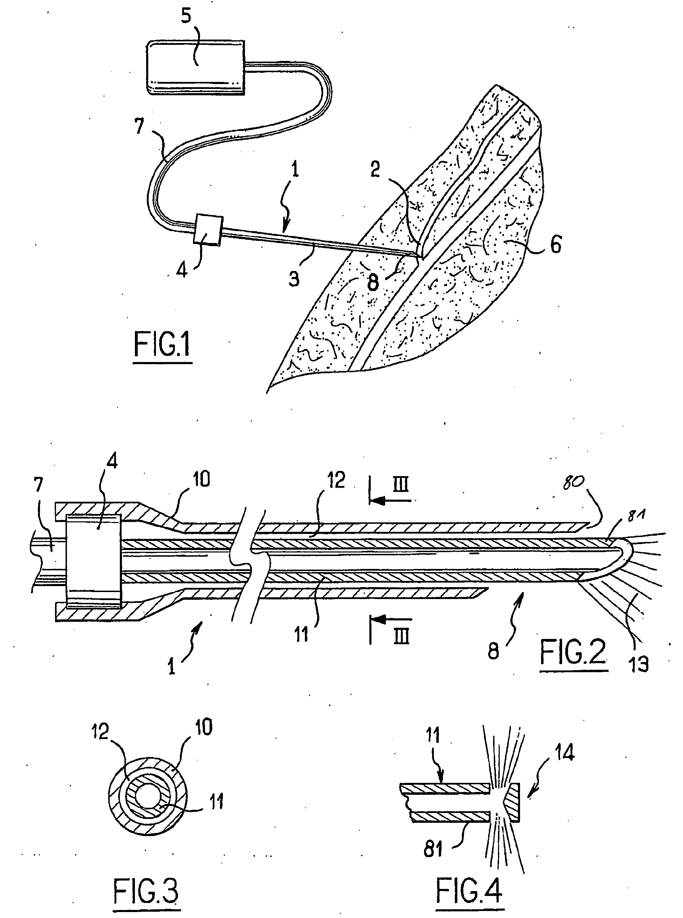

La figure 1 illustre très schématiquement l'utilisation d'un instrument 1 selon l'invention pour le traitement d'une varice 2. L'instrument a la forme d'une aiguille 3 contenant un guide pour de la lumière laser. Le contact du guide avec l'embout de l'aiguille peut être de forme conique afin de s'y emboîter de manière cohérente. L'Instrument comprend des moyens de raccord 4 pour une source 5 de lumière laser. Un câble optique 7 relie la source laser 5 et les moyens de raccord 4. Dans la position illustrée, l'aiguille 3 a été piquée dans un membre inférieur 6. Une extrémité distale 8 de l'aiguille, c'est-à-dire son extrémité la plus éloignée de la source de lumière 5, est introduite dans une extrémité de la varice 2. Un tir laser peut alors être effectué au travers de l'extrémité distale 8 de l'aiguille afin de provoquer la sclérose de l'extrémité de la varice, empêchant par la suite l'afflux de sang dans la varice. Plusieurs tirs lasers peuvent être réalisés le long de la varice traitée, environ tous les deux mm tout en retirant progressivement l'aiguille. Des repères peuvent alors être prévus sur une gaine de l'aiguille.

Les figures 2 et 3 illustrent un premier mode de réalisation pour un instrument 1 selon l'invention. Dans ce premier mode de réalisation, l'instrument comprend une aiguille 10 tubulaire. L'aiguille 1 0 est biseautée au droit de l'extrémité distale 8 de l'instrument 1. L'instrument comprend en outre une fibre optique 11, elle aussi biseauté au droit de l'extrémité distale 8. L'aiguille 10 forme une gaine tubulaire pour la fibre optique 11. A l'extrémité distale 8 de l'instrument 1, l'extrémité distale 81 biseautée de la fibre optique 11 s'étend au-delà de l'extrémité distale 80 biseautée de J'aiguille 10 afin de laisser diffuser la lumière laser.

L'instrument comprend à son extrémité proximale, des moyens de raccord 4 à un câble optique 7 le reliant à une source laser, non représentée aux figures 2 et 3.

Un espace 12 formant chenal entre l'aiguille et la fibre peut être utilisé pour injecter un anesthésiant, avant le tir laser. Cet espace peut aussi être utilisé pour vérifier que l'extrémité distale 8 est bien positionnée, le reflux de sang dans l'aiguille en étant un indice.

L'instrument de la figure 2 est prévu pour assurer une diffusion frontale de la lumière, c'est-à-dire sensiblement dans l'axe de la fibre optique 12. Le biseau à l'extrémité de la fibre permet cependant de déporter latéralement la diffusion d'une partie de la lumière.

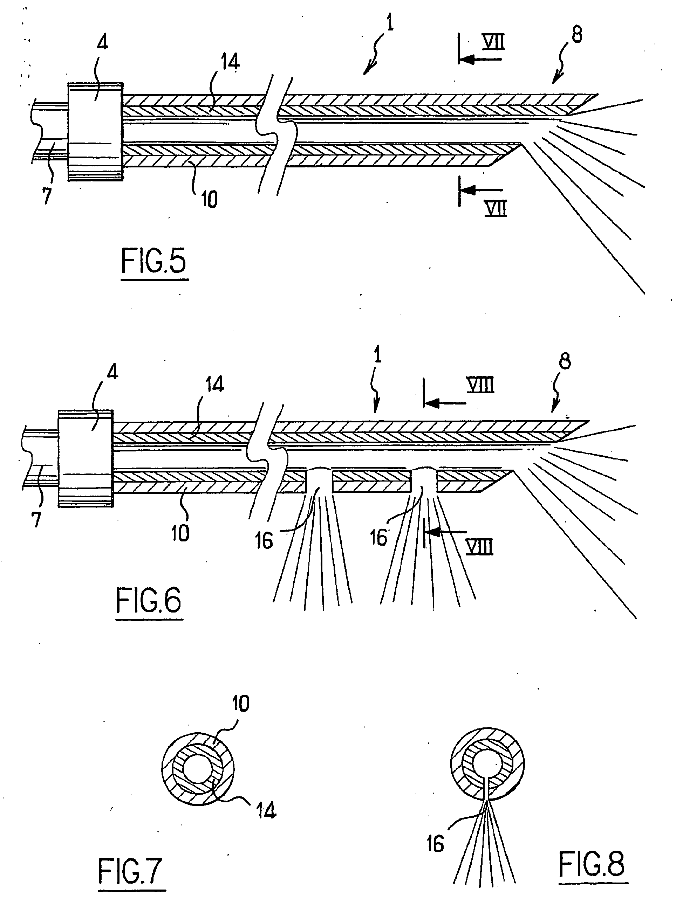

Comme illustré à la figure 4 dans un deuxième mode de réalisation, il est prévu, à l'extrémité distale 81 de la fibre optique, des moyens 14 de diffusion latérale de la lumière. Ceux-ci permettent un traitement circonférentiel plus aisé. Dans l'exemple de la figure 4, de tels moyens de diffusion latérale interdit complètement la diffusion frontale de fa lumière. Il est aussi possible de prévoir des moyens permettant à la fois une diffusion frontale et latérale de la lumière, par exemple tels qu'illustrés aux figures 6 et 8 décrites plus bas.

On va maintenant décrire un troisième mode de réalisation pour un instrument selon l'invention, en référence aux figures 5 et 7, seulement en ce qu'il diffère de celui de la figure 2.

A la figure 5, le guide de lumière 14 n'est pas une fibre optique indépendante de l'aiguille. Ici le guide de lumière est réalisé par un habillage 14 de l'intérieur de l'aiguille 1 O. Cet habillage est solidaire de l'aiguille sur toute la surface ainsi habillée. Etant en silice, il présente les mêmes qualités de guidage de la lumière qu'une fibre optique. Il bénéficie en outre de la protection de la gaine que forme l'aiguille 10, notamment en terme de solidité et de protection aux chocs. De plus, l'habillage étant solidaire de l'aiguille, la précision de l'instrument est augmentée.

Le biseau à l'extrémité distale 8 de l'instrument affecte de façon identique l'aiguille et son habillage, les extrémités distales du guide de lumière et de l'aiguille étant sensiblement confondues.

Les figures 6 et 8 illustrent un quatrième mode de réalisation, variante du mode de réalisation de la figure 5. Dans ce mode de réalisation, outre l'ouverture permettant une diffusion sensiblement frontale de la lumière au travers de l'extrémité distale 8, l'instrument 1 comprend deux fenêtres latérales 16. Ces fenêtres sont formées transversalement au travers de la paroi de l'aiguille et au travers de son habillage, à distance de l'extrémité distale 8. Les fenêtres sont disposées sensiblement alignées selon une génératrice du cylindre formé par l'aiguille. De telles fenêtres permettent des diffusions latérales et frontale simultanées de la lumière. Leurs dispositions peuvent être choisies en fonction notamment de la zone à traiter.

Bien sûr, l'invention n'est pas limitée aux exemples qui viennent d'être décrits et de nombreux aménagements peuvent être apportés à ces exemples sans sortir du cadre de l'invention.

Ainsi, notamment pour une plus grande précision, il peut être avantageux que le guide de lumière, par exemple une fibre optique se prolonge au-delà du support, par exemple une aiguille. Il peut être prévu que la fibre dépasse de 0 à 1 cm, de préférence d'environ 5 mm. Pour protéger la fibre optique au cours de d'une ponction, il peut être prévu des moyens pour rétracter la fibre dans l'aiguille et des moyens pour étendre la fibre optique au-delà de l'extrémité distale de l'aiguille, jusque dans une position de travail.

Il peut aussi être prévu des moyens de rotation du guide de lumière relativement à d'autres parties de l'instrument ; par exemple relativement au support, notamment dans le cas d'une aiguille contenant une fibre optique, ou relativement à des moyens de raccord, notamment dans le cas d'une aiguille intérieurement habillée de silice. Ces moyens de rotation permettent ainsi un traitement circonférentiel facilité.

Aussi, un instrument selon l'invention peut être utilisé à d'autres applications vasculaires, par exemple la sclérose de varices non seulement sur les membres inférieures, mais aussi de varices pelviennes ou oesophagiennes, d'hémorroïdes, ou la sclérose vasculaire lors d'opérations chirurgicales. On peut aussi utiliser un instrument selon l'invention pour réaliser une perforation tissulaire, ou comme alternative aux colles biologiques, de type cyanoacrylates, pour toute embolisation intra-vasculaire, notamment pour les fistules congénitales ou acquises, ou veineuse, notamment pour des malformations ou des angiomes.

D'autres applications existent, notamment pour la destruction tissulaire, particulièrement si l'on veut une grande précision. Ainsi, en dermatologie, l'utilisation d'un laser de la longueur d'onde de 980nm, ou de longueurs d'onde des Erbiums et des Holmiums, 2µm environ, absorbées par l'eau, rend un instrument selon l'invention particulièrement efficace, notamment pour le traitement des condylomes, verrues et autres lésions cutanées. Les cicatrices sont de qualité supérieure à celles obtenues par d'autres techniques.

Dans le cadre de la destruction cellulaire, d'autres applications peuvent aussi être la détersion tissulaire en mode contact, par exemple appliquée aux ulcères. Un instrument selon l'invention peut encore être utilisé pour la destruction de métastases tissulaires, par exemple intra-hépathique, plus ergonomique et moins coûteuse que par radiofréquence. La destruction tissulaire par laser implique un phénomène d'absorption tissulaire des photons avec conversion secondaire en chaleur. Cette absorption étant ciblée à certains constituants moléculaires spécifique au tissu (chromophores), l'effet thermique est plus spécifique qu'un autre procédé de chauffage thermique des tissus tels que la radiofréquence par exemple. Cette spécificité permet de nouvelles applications des lasers comme la photothérapie dynamique en cancérologie par exemple.

L'utilisation d'un support rigide pour le guide de lumière permet une utilisation plus précise et une maniabilité supérieure. Ainsi, les lésions occasionnées par le traitement sont plus précises. La stimulation biologique améliore la cicatrisation. L'hémorragie est réduite, ainsi que le risque infectieux, la carbonisation impliquant des températures voisines ou supérieures à 350°C ou 400°C, ce qui correspond à l'incandescence tissulaire. Les douleurs post-opératoires sont elles aussi réduites, notamment dans le cas des détersions d'ulcères ; II y a moins d'infections post-opératoires. II existe une inhibition secondaire de l'inflammation et des facteurs algogènes au niveau des terminaisons nerveuses.

En outre, des paramètres peuvent être réglables, par exemple la longueur d'onde, le temps d'émission et de pose entre chaque tir, la fluence, l'irradiance, le mode continu ou impulsionnel (simple ou multiple) ou l'aire de destruction selon la diffusion optique à l'extrémité du guide de lumière.

Le guide de lumière peut être en un autre matériau que la silice, selon la longueur d'onde de laser.

## Revendications

1. Instrument médical (1) comprenant un support formé par une aiguille (3) rigide tubulaire qui réalise une gaine dans laquelle est monté un guide de lumière (11) comportant un orifice de diffusion à son extrémité distale (81), le guide de lumière étant relié à une source de lumière (5) destinée à interagir avec un élément vasculaire (2) et/ou cellulaire, **caractérisé en ce que** le guide de lumière pouvant dépasser l'extrémité distale (80) du support d'au plus 1 cm comporte à son extrémité ou à proximité de son extrémité distale (81) nue, au moins une fenêtre latérale formant un moyen de diffusion de lumière permettant un traitement circonférentiel par une diffusion déportée latéralement d'au moins une partie de la lumière.

2. Instrument (1) selon la revendication 1, **caractérisé en ce que** le guide de lumière (11) comprend un habillage (14) d'une paroi intérieure de l'aiguille (3).

3. Instrument (1) selon la revendication 2, **caractérisée en ce que** l'habillage (14) est en silice.

4. Instrument (1) selon l'une des revendications 1 à 3, **caractérisé en ce que** le guide de lumière (11) comprend une fibre optique.

5. Instrument (1) selon l'une des revendications 1 à 4, **caractérisé en ce qu'**il comprend des moyens pour une émission frontale de la lumière.

6. Instrument (1) selon les revendications 3 et 5, **caractérisé en ce que** les moyens pour une émission frontale de la lumière comprennent une extrémité biseautée (80) de l'aiguille (10) et/ou (81) du guide de lumière (11).

7. Instrument (1) selon l'une des revendications 1 à 6, **caractérisé en ce qu'**il comprend des moyens (16) pour une émission latérale de la lumière.

8. Instrument (1) selon l'une des revendications 1 à 7, **caractérisé en ce qu'**il comprend des moyens de rotation du guide de lumière (11).

9. Instrument (1) selon l'une des revendications 1 à 8, **caractérisé en ce qu'**il comprend des moyens d'extension du guide de lumière (11) au-delà d'une extrémité distale (80) du support.

10. Instrument selon l'une des revendications 1 à 9, **caractérisé en ce qu'**il comprend des moyens pour injecter un anesthésique.

11. Instrument selon l'une des revendications 1 à 10, **caractérisé en ce qu'**il comprend des moyens pour vérifier le bon positionnement de l'instrument.

12. Système comprenant un instrument selon l'une des revendications précédentes, **caractérisé en ce qu'**il comprend une source (5) de lumière laser.

13. Système selon la revendication 12, **caractérisé en ce que** la longueur d'onde de la lumière est comprise entre 800 et 980 nm.

## Patentansprüche

1. Medizinisches Instrument (1), welches eine Stütze aufweist, welche durch eine feste, rohrförmige Nadel (3) gebildet ist, welche eine Hülle bildet, in welcher ein Lichtleiter (11) montiert ist, welcher an seinem distalen Ende (81) eine Streuöffnung aufweist, wobei der Leichtleiter mit einer Lichtquelle (5) verbunden ist, welche dazu bestimmt ist, mit einem Bestandteil (2) eines Gefäßsystems und/oder einer Zelle zu wechselwirken, **dadurch gekennzeichnet, dass** der Lichtleiter, welcher über das distale Ende (80) der Stütze um mehr als 1 cm hinausragen kann, an seinem freiliegenden distalen Ende (81) oder in der Nähe seines freiliegenden distalen Endes (81) mindestens ein seitliches Fenster aufweist, welches ein Lichtstreumittel bildet, welches eine Behandlung in Umfangsrichtung ermöglicht mittels einer lateral abgegebenen Streuung mindestens eines Teils des Lichts.

2. Instrument (1) gemäß Anspruch 1, **dadurch gekennzeichnet, dass** der Lichtleiter (11) eine Wandverkleidung (14) innerhalb der Nadel (3) aufweist.

3. Instrument (1) gemäß Anspruch 2, **dadurch gekennzeichnet, dass** die Verkleidung (14) aus Siliziumdioxid ist.

4. Instrument (1) gemäß einem der Ansprüche 1 bis 3, **dadurch gekennzeichnet, dass** der Lichtleiter (11) eine optische Faser aufweist.

5. Instrument (1) gemäß einem der Ansprüche 1 bis 4, **dadurch gekennzeichnet, dass** es Mittel zur frontalen Emission des Lichts aufweist.

6. Instrument (1) gemäß den Ansprüchen 3 und 5, **dadurch gekennzeichnet, dass** die Mittel für eine frontale Lichtemission ein abgeschrägtes Ende (80) der Nadel (10) und/oder (81) des Lichtleiters (11) aufweisen.

7. Instrument (1) gemäß einem der Ansprüche 1 bis 6, **dadurch gekennzeichnet, dass** es Mittel (16) für eine seitliche Emission des Lichts aufweist.

8. Instrument (1) gemäß einem der Ansprüche 1 bis 7, **dadurch gekennzeichnet, dass** es Mittel zur Drehung des Lichtleiters (11) aufweist.

9. Instrument (1) gemäß einem der Ansprüche 1 bis 8, **dadurch gekennzeichnet, dass** es Mittel zur Verlängerung des Lichtleiters (11) über das distale Ende (80) der Stütze hinaus aufweist.

10. Instrument (1) gemäß einem der Ansprüche 1 bis 9, **dadurch gekennzeichnet, dass** es Mittel zum Injizieren eines Anästhetikums aufweist.

11. Instrument (1) gemäß einem der Ansprüche 1 bis 10, **dadurch gekennzeichnet, dass** es Mittel zum Bestätigen der guten Positionierung des Instruments aufweist.

12. System, welches ein Instrument gemäß einem der vorhergehenden Ansprüche aufweist, **dadurch gekennzeichnet, dass** es eine Laserlichtquelle (5) aufweist.

13. System gemäß Anspruch 12, **dadurch gekennzeichnet, dass** die Wellenlänge des Lichts zwischen 800 und 980 nm liegt.

## Claims

1. Medical instrument (1) comprising a support formed by a needle (3), said support forming a rigid tubular sheath which is mounted in a light guide (11) having a transmission orifice at its distal end (81), the light guide being connected to a light source (5) for interacting with a cellular and/or vascular element (2), **characterized in that** the light guide, that may protrude the distal end (80) of the carrier at the most by 1 cm, comprises at its end or in proximity to its opened distal end (81) at least one lateral window forming means for light diffusion allowing a circumferential treatment thanks to a laterally deported diffusion of at least part of the light.

2. Instrument according to claim 1, **characterized in that** the light guide (11) comprises a coating (14) of an inner wall of the needle (3).

3. Instrument according to claim 2, **characterized in that** the coating (14) is a silica coating.

4. Instrument according to one of claims 1 to 3, **characterized in that** the light guide (11) comprises an optical fibre.

5. Instrument according to one of claims 1 to 4, **characterized in that** it comprises means for frontal emission of light.

6. Instrument according to one of claims 3 to 5, **characterized in that** means for frontal emission of light comprise a bevelled end (80) of the needle (10) and/or (81) of the light guide (11).

7. Instrument according to any of claims 1 to 6, **characterized in that** it comprises means (16) for lateral emission of light.

8. Instrument according to any of claims 1 to 7, **characterized in that** it comprising means for rotating the light guide (11).

9. Instrument according to any of claims 1 to 8, **characterized in that** it comprises extension means for the light guide (11) beyond a distal end (80) of the support.

10. Instrument according to any of claims 1 to 9, **characterized in that** it comprises resources to inject an anesthetic.

11. Instrument according to any of claims 1 to 10, **characterized in that** it comprises resources to verify the proper positioning of the instrument.

12. System comprising an Instrument according to any of the preceding claims, **characterized in that** it comprises a laser light source (5).

13. System according to claim 12, **characterized in that** the light wavelength lies between 800 and 980 nm.
